# EUROPÄISCHE PATENTSCHRIFT

(11) **EP 1 098 982 B1**
(45) Veröffentlichungstag und Bekanntmachung des Hinweises auf die Patenterteilung: **14.04.2004**
(21) Anmeldenummer: 99947198.0
(22) Anmeldetag: 14.07.1999
(51) Int. Cl.: C12N 15/54, C12N 15/82, C12N 9/10, C12P 19/44

(54) **VERWENDUNG EINES GLUCOSYLGLYCEROLPHOSPHATSYNTHASEGENS ZUR ERZEUGUNG DER NIEDERMOLEKULAREN SCHUTZSUBSTANZ GLUCOSYLGLYCEROL IN PFLANZEN**
USE OF GLUCOSYLGLYCEROL PHOSPHATE SYNTHASE GENE FOR PRODUCING THE LOW-MOLECULAR PROTECTIVE SUBSTANCE GLUCOSYLGLYCEROL IN PLANTS
UTILISATION DU GENE DE GLUCOSYLGLYCEROLPHOSPHATE SYNTHASE POUR LA PRODUCTION DE GLUCOSYLGLYCERINE FAIBLEMENT MOLECULAIRE COMME SUBSTANCE DE PROTECTION DANS LES PLANTS

(30) Priorität: 17.07.1998 DE 19832334
(43) Veröffentlichungstag der Anmeldung: 16.05.2001
(73) Patentinhaber: Norika-Nordring-Kartoffelzucht- und Vermehrungs GmbH., 18190 Gross Lüsewitz (DE)
(72) Erfinder: HAGEMANN, Martin, D-18057 Rostock (DE)
(74) Vertreter: Bauer, Wulf, Dr.
(86) Internationale Anmeldenummer: PCT/DE1999/002150
(87) Internationale Veröffentlichungsnummer: WO 2000/004164

(56) Entgegenhaltungen:
- WO-A-96/00789
- WO-A-97/42326
- KANEKO T ET AL: "Sequence analysis of the genome of the unicellular cyanobacterium Synechocystis sp. strain PCC6803. II. Sequence determination of the entire genome and assignment of potential protein-coding regions" DNA RESEARCH,JP,UNIVERSAL ACADEMY PRESS, Bd. 3, Nr. 3, 1. Januar 1996 (1996-01-01), Seiten 109-136, XP002084893 ISSN: 1340-2838 & "AC D90913; P74258" EBI DATBASE,1997,
- KAUSHAL G P ET AL: "Plant glucosidase II catalyzes a transglucosylation reaction in addition to the hydrolytic reaction." ARCHIVES OF BIOCHEMISTRY AND BIOPHYSICS, (1989 AUG 1) 272 (2) 481-7. , XP000889912
- HAGEMANN MARTIN ET AL: "Activation and pathway of glucosylglycerol synthesis in the cyanobacterium Synechocystis sp. PCC 6803." MICROBIOLOGY (READING) 1994, Bd. 140, Nr. 6, 1994, Seiten 1427-1431, XP000889909
- HAGEMANN MARTIN ET AL: "The stpA gene from Synechocystis sp. strain PCC 6803 encodes the glucosylglycerol-phosphate phosphatase involved in cyanobacterial osmotic response to salt shock." JOURNAL OF BACTERIOLOGY 1997, Bd. 179, Nr. 5, 1997, Seiten 1727-1733, XP002132733 ISSN: 0021-9193
- HAGEMANN, MARTIN ET AL: "NaCl acts as a direct modulator in the salt adaptive response. Salt-dependent activation of glucosylglycerol synthesis in vivo and in vitro" J. PLANT PHYSIOL. (1996), 149(6), 746-752 , XP000890019
- JOSET FRANCOISE ET AL: "Dynamics of the response of cyanobacteria to salt stress: Deciphering the molecular events." PHYSIOLOGIA PLANTARUM 1996, Bd. 96, Nr. 4, 1996, Seiten 738-744, XP000890018 ISSN: 0031-9317
- MARIN KAY ET AL: "The ggpS gene from Synechocystis sp. strain PCC 6803 encoding glucosyl-glycerol-phosphate synthase is involved in osmolyte synthesis." JOURNAL OF BACTERIOLOGY SEPT., 1998, Bd. 180, Nr. 18, September 1998 (1998-09), Seiten 4843-4849, XP002132734 ISSN: 0021-9193
- Trends in plant science (1999) 4: 315
- Journal of plant physiology (1998) 152: 525
- Plant physiol. (1997) 113: 181
- Physiologia Plantarum (1993) 97: 223-226

## Beschreibung

Der Ertrag landwirtschaftlicher Nutzpflanzen ist stark von Faktoren der Umwelt abhängig. Häufig treten Perioden auf, in denen die Wachstumsbedingungen stark von optimalen Verhältnissen abweichen und damit auf die Pflanzen einen akuten oder auch chronischen Streß ausüben. Durch Streßsituationen wird in den Pflanzen eine Anpassungsreaktion (nichtgenetische Anpassung - Akklimation) ausgelöst, die eine Optimierung des Stoffwechsels in Bezug auf die veränderten Umweltbedingungen zum Ziel hat. Das Anpassungsvermögen einer Pflanze ist dabei genetisch determiniert und gestattet somit nur eine artspezifisch-beschränkte Flexibilität in der Reaktionsbreite.

Zu den häufigsten Umweltstressoren gehören Trockenheit, erhöhte Salzgehalte, Kälte und oxidativer Streß. Die ersten 3 Stressoren führen analog zu einer Verminderung des zellulären Wassergehalts, so daß Übereinstimmungen in der Anpassungsstrategie nicht verwundern. Oxidativer Streß kann durch vielfältige Schädigungen des Photosyntheseapparates hervorgerufen werden, neben den oben genannten Umweltstressoren sind vor allem zu hohe Lichtintensitäten und Luftschadstoffe zu nennen. Bezüglich der Trocken- und Salztoleranz werden Pflanzen in Glykophyten mit einer geringen Resistenz sowie Halophyten (hohe Salztoleranz) bzw. Xerophyten (hohe Trockentoleranz) eingeteilt. Die weitaus meisten der heute genutzten Kulturpflanzen sind ausgesprochene Glykophyten, mit einer sehr geringen Resistenz gegenüber Wassermangel und damit gekoppelten erhöhten Salzgehalten im Boden.

Eine weitverbreitete Strategie bei der Anpassung an ungünstige Umweltbedingungen besteht in der Akkumulation von niedermolekularen Schutzsubstanzen (compatible solutes) in Pflanzen sowie Mikroorganismen. Diese Substanzen erhöhen das interne osmotische Potential, wirken damit einem Wasserverlust entgegen, treten in Wechselwirkungen mit Makromolekülen, vermindern damit deren Anfälligkeit gegenüber Denaturierungen, und können mit Sauerstoffradikalen reagieren, womit ein wesentlicher schädigender Faktor nach oxidativem Streß bekämpft wird. Compatible solutes sind niedermolekulare organische Stoffe, die sehr hydrophil sind und keine Nettoladung aufweisen. Um eine Akkumulation großer Mengen zu erreichen, stellen diese Stoffe oft Endpunkte von Biosynthesewegen dar, so daß kein oder nur ein sehr genau regulierter Abbau erfolgen kann. Bisher wurden ca. 30 derartige Substanzen bei Pflanzen und Mikroorganismen gefunden. Es besteht eine sehr gute Korrelation zwischen dem Gehalt dieser Substanzen und der erreichten Salz- bzw. Trockentoleranz. Um eine Wirkung auszuüben, ist eine Akkumulation in relativ hohen Konzentrationen notwendig (10 - 500 mM). Daher werden diese Stoffe in Pflanzen ausschließlich in den metabolisch aktiven Kompartimenten (Cytoplasma, Kern, Plastiden, Mitochondrien etc.) angereichert, während in den metabolisch inaktiveren Teilen der Pflanze (Vacuole, Zellwandraum) Ionen akkumuliert werden.

Entscheidend für die Schutzwirkung dieser Stoffe sind drei Faktoren: (i) Die Menge der compatible solutes muß dem Grad der äußeren Belastung entsprechend so eingestellt werden, daß die Pflanze gegenüber dem Boden hyperosmotisch ist und somit Wasser aufnehmen kann, wobei der Gehalt in allen Pflanzenteilen abgestimmt erhöht wird. (ii) Die chemische Natur der compatible solutes führt zu graduell verschiedenen Resistenzerhöhungen, wobei offensichtlich nicht die osmotische sondern die verschieden stark ausgeprägte direkte Schutzkomponente für Makromoleküle entscheidend ist. (iii) Die compatible solutes sollten im Cytoplasma und den darin befindlichen Organellen konzentriert werden, während Ionen vor allem in der Vacuole sowie im Apoplasten kompartimentiert vorliegen müssen.

Die Steigerung der Salz- und Trockentoleranz stellt seit Jahren ein wichtiges Ziel in der Pflanzenzüchtung dar. Bisher wurde ausgehened von relativ salztoleranten Pflanzen eine Selektion höher resistenterer Sorten angestrebt, wobei neben den Ganzpflanzen auch Gewebe - und Zellkulturen zum Einsatz kamen. Als ein Parameter im Screening wurde der interne Gehalt an compatible solutes herangezogen, um schon früh zu einer Aussage über eine potentielle Steigerung der Salztoleranz zu kommen. Bisher ist es jedoch nicht gelungen, auf diesem Wege zu deutlich verbesserten Sorten zu kommen. Unter den in Kulturpflanzen natürlich vorkommenden compatible solutes werden häufig Saccharose und Prolin gefunden. Diese beiden Stoffe sind jedoch Bestandteil des aktiven pflanzlichen Stoffwechsels, so daß eine Steigerung der streßproportionalen Akkumulation schwer zu erreichen ist, da sie auch einem starken Abbau und Umbau unterlegen.

In der Literatur wird daher der Weg favorisiert, durch Übertragung von Genen, die Enzyme für bisher pflanzenfremde Synthesewege von compatible solutes kodieren, schneller und erfolgreicher zu einer Erhöhung der Salz- bzw. allgemeinen Streßtoleranz zu kommen. Das ist in Laboruntersuchungen auch durch eine Übertragung von Genen, die Enzyme für die Mannitol-, Glycinbetain-, Onitol- bzw. Trehalosesynthese in Mikroorganismen bzw. halophilen Pflanzen kodieren, gelungen. Allerdings sind die erreichten Steigerungen der Toleranz eher gering, was an der nicht ausreichenden akkumulierten Menge sowie auf der nichtvorhandenen Regulierbarkeit liegen könnte.

Die Erfindung hat es sich zur Aufgabe gemacht, die compatible Substanz Glucosylglycerol in Nutzpflanzen direkt herzustellen und speichern zu lassen. Ziel ist also insbesondere die Herstellung gentechnisch veränderter Nutzpflanzen mit einer erhöhten Resistenz gegenüber Salz-, Trocken-, Kälte-, Hitze- und oxidativem Streß, einer verbesserten Lagerfähigkeit sowie einem erhöhten Anteil von biologisch abbaubaren Kohlenhydraten im Lebensmittel.

Die Aufgabe wird gelöst durch Anspruch 1 und die weiteren Ansprüche.

Die Erfindung betrifft somit die Verwendung eines Glucosylglycerolphosphatsynthasegens welches für ein Protein mit einer Aminosäuresequenz codiert, die zu mehr als 60%, 70%, vorzugsweise mit mehr als 80% und insbesondere mit mehr als 90% identisch zu einer Aminosäuresequenz ist, die aus der in Tabelle 1 dargestellten DNA Sequenz abgeleitet ist sowie die Verwendung des genannten Proteins zur Erzeugung von Glucosylglycerol in kulturpflanzen. Ebenso betrifft die Erfindung transgene Nutzpflanzen, insbesondere Nahrungs- und Futtermittelpflanzen sowie Pflanzen der sogenannten nachwachsenden Rohstoffe, welche ein übertragenes Glucosylglycerol phosphatsynthesegen wie oben definiert tragen. Die Erzeugung dieser Pflanzen kann unter Einsatz konstitutiver und regulierter Promotoren und Signalsequenzen zum Transport des Proteins in Organellen erfolgen. In diesen Pflanzen führt die Expression der Gene zur
A) spezifischen Anreicherung der niedermolekularen Schutzsubstanz Glucosylglycerol
B) Erhöhung der Streßresistenz
C) Verbesserung des Ertrages, des Nährwertes und der Lagerfähigkeit.

Durch Modifikation der Aminosäuresequenz der Glucosylglycerol-phosphat-synthase kann die Regulation der Aktivität des Enzyms in Abhängigkeit vom Salzgehalt verändert werden.

Die Übertragung der Glucosylglycerol-phasphatsynthasegene kann in den transgenen Pflanzen zu einer Akkumulation des Glucosylglycerol-phosphates führen, wenn es in diesen Pflanzen keine ausreichend aktiven unspezifischen Zuckerphosphatasen geben sollte. Auf Grund der Ladung ist Glucosylglycerol-phosphat wenig kompatibel mit dem Stoffwechsel. Dieses Problem kann durch die Expression eines Glucosylglycerol-phosphatphosphatase-gens (*stpA*) aus *Synechocystis* sp. PCC 6803 in der transgenen Pflanze aufgehoben werden.

Cyanobacterien, aus deren Gruppe die Vorläufer der Chloroplasten stammen, bilden die niedermolekulare Schutzsubstanz Glucosylglycerol (GG, 2-O-(α-D-glucopyranosyl)-glycerol). Sie ist vor allem bei euryhalinen Cyanobacterien verbreitet und gestattet ein Wachstum in salzhaltigen Medien (bis 1,5 M NaCl). In salzadaptierten Zellen kann das akkumulierte. GG bis zu 20% der Trockenmasse ausmachen. Die Substanz liegt im Cytoplasma der Zelle frei gelöst vor. GG hat eine Molmasse von 254 g/Mol und gehört zur Gruppe der Heteroside, das heißt an der Kohlenhydratuntereinheit Glucose liegt mit Glycerol ein Polyol gebunden vor.

Neben der Vermittlung einer hohen Salzresistenz konnte gezeigt werden, daß eine GG-Akkumulation eine erhöhte Thermo- und Starklichttoleranz hervorruft (systemische Resistenz). Ein gezieltes Ausschalten der Glucosylglycerolsynthese zieht einen kompletten Verlust der Salztoleranz nach sich und auch die systemische Resitenz wird nicht mehr ausgebildet. Diese Substanz ist ebenfalls in einigen Wildpflanzen in geringer Konzentration vorhanden, wo sie bei der Trockentoleranz eine Rolle spielen soll. Die Untersuchungen der Genetik und Biochemie der Glucosylglycerolsynthese führten zur Klonierung des Gens, das für das Schlüsselenzym der Biosynthese, die Glucosylglycerol-Phosphat-Synthase, kodiert. Dieses Gen wurde in *Escherichia coli* überexprimiert, wo die erhoffte biochemische Aktivität nachgewiesen wurde.

Ein erfindungsgemäßes Glucosylglycerol-phosphat-synthasegen wurde aus *Synechocystis* sp. PCC 6803 oder *Synechococcus* sp. PCC 7002 isoliert. Nach Mutationsanalyse der Gene konnte die DNA Basen-Sequenz aus der Cyanobase-Datenbank entnommen werden. Der bisher einer anderen Funktion zugeordnete Leserahmen sll 1566 (Tabelle 1) konnte als Glucosylglycerol-phosphat-synthasegen (*ggpS*) in *Synechocystis* sp. PCC 6803 identifiziert werden.

Glucosylglycerol wird erfindungsgemäß nach Übertragung dieser Gene in Nutzpflanzen direkt hergestellt und gespeichert. Glucosylglycerol ist eine effektive niedermolekulare Schutzsubstanz. Seine Synthese und Akkumulation soll zu einer Verbesserung der Toleranz gegenüber abiotischen Stressoren, insbesondere Trockenheit, erhöhte Salzgehalte, Kälte und oxidativem Streß führen. Gegenüber anderen potentiell wirksamen niedermolekularen Schutzsubstanzen weist Glucosylglycerol Vorteile auf. Es handelt sich um eine Stickstofffreie Schutzsubstanz, so daß die Stickstoffbilanz der Pflanze nicht negativ beeinflußt wird. Es ist ein reines Heterosid, das in dieser Form nicht in Pflanzen vorkommt. Daher sollte es im Chloroplasten bzw. im Cytoplasma der produzierenden Gewebe verbleiben und dort seine erwünschte Schutzwirkung entfalten, während in der Vakuole unerwünschte toxische Ionen gespeichert werden. Nicht zuletzt sollte es keine abbauende Aktivität in den Pflanzen geben, so daß schon eine geringen biochemische Aktivität zu einer relevanten Akkumulation und Schutzwirkung führt.

Die Anreicherung von Glucosylglycerol in den Pflanzen führt zu einer verbesserten Wasseraufnahme und -rückhaltung in den Pflanzen sowie zu insgesamt streßresistenteren Nutzpflanzen. Dadurch werden bei schlechter und unregelmäßiger Wasserversorgung Einbußen im Ertrag vermindert. Es können Böden landwirtschaftlich genutzt werden, die durch erhöhte Salzgehalte zuvor nicht oder nur sehr eingeschränkt nutzbar waren. Die Erhöhung der Streßtoleranz kann den Anbau bestimmter Nutzpflanzen in bisher aus klimatischen und/oder Bodenbeschaffenheitsgründen nicht genutzten Gegenden gestatten, eine Verlängerung der Vegetationsperiode in die trockenere Jahreszeit hinein sowie eine Verminderung des Bewässerungsaufwandes hervorrufen. Insgesamt ist mit stabileren Erträgen unter ungünstigen Klimaten zu rechnen. Auf diese Weise werden höhere Erträge bei geringeren agronomischen Aufwendungen möglich.

Im Kartoffelanbau führt die Akkumulation von GG zu einem weiteren Aspekt. Durch die verbesserte Wasserbindung wird die Lagerfähigkeit der Knollen verbessert, da sie länger in Form bleiben und mechanischen Schädigungen widerstehen. Bisher wurde dieses durch erhöhtes Ausbringen von Kalidünger erreicht. Diese kostenintensive Düngung könnte in GGakkumulierenden Pflanzen zusätzlich eingeschränkt werden. Darüberhinaus kann eine verlängerte Lagerung der Kartoffeln dazu beitragen, teure Kartoffelimporte und Verarbeitungungspausen in der kartoffelverarbeitenden Industrie zu vermeiden.
In der Zeichnung zeigen:
Fig. 1 die Strukturformel von Glycosylglycerol (GG),
Fig. 2 ein Säulendiagramm für den Glycosylgehalt in µg/ml/E750, wobei E750 die optische Dichte bei 750 nm ist, für die Zellen eines Wildtyps (schwarze Säulen), die Zellen der Mutante GK2 (offene Säulen) und Zellen der Mutante GK2, denen GG im Medium zugegeben wurde (gestrichelte Säulen), für unterschiedliche Medien, deren Salzgehalt in %-NaCl jeweils hinter dem Schrägstrich angegeben ist und
Fig. 3 ein Säulendiagramm der Photosyntheseaktivität in Prozent relativ, zur Kontrolle des Wildtyps für dieselben Zellen wie in Fig. 2.

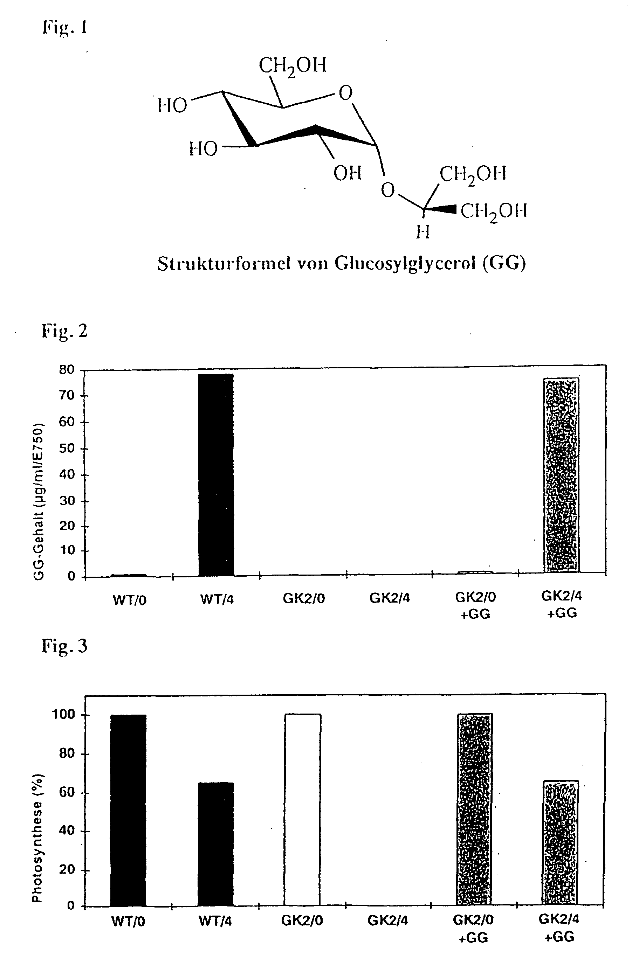

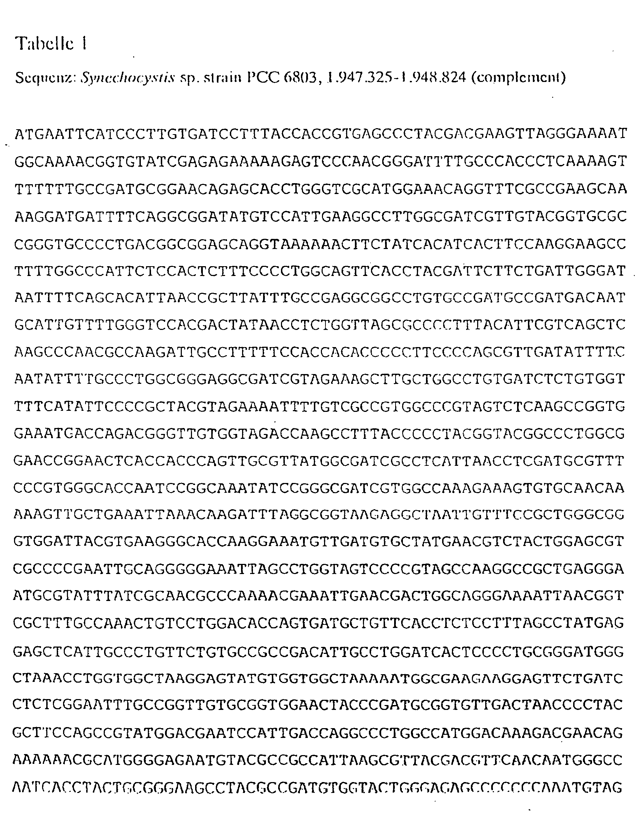

Folgende Versuche wurden gemacht, um die Erfindung praktisch zu erproben. Vor der Übertragung in Pflanzen konnte die Schutzwirkung von Glucosylglycerol (GG) am mikrobiellen Modell *Synechocystis* sp. PCC 6803 zweifelsfrei nachgewiesen werden (siehe Fig. 2 und 3). In Zellen des Wildtyps (WT) dieses Cyanobacteienstammes, die an hohe Salzgehalte (4% NaCl = WT/4) angepaßt waren, wird GG in hohen Konzentrationen (bis 80 µg GG/ml/E750) nachgewiesen, während unter salzfreien Bedingungen aufgezogene Kontrollzellen (WT/0) nur sehr wenig GG enthalten (Fig. 2). Die GG-Akkumulation gstattet es den Zellen offensichtlich, sich an die hohe Salzbelastung anzupassen, so daß sie weiterhin Photosynthese betreiben können (Fig. 3).

Der Nachweis der Schutzwirkung von Glucosylglycerol erfolgt mit Mutanten des Cyanobacteriums *Synechocystis* sp. PCC 6803. Es wurde die Akkumulation von Glucosylglycerol (GG) in der Biomasse (Fig. 2), und die Photosyntheseaktivität (Fig. 3) in Zellen des Wildtyps (WT, schwarze Säulen) sowie in der Mutante GK2 (offene Säulen), die ein defektes *ggpS*-Gen aufweist, gemessen. Die Zellen des WT und der Mutante GK2 wurden in salzarmem Kontrollmedium (WT/0 und GK2/0) bzw. in 4%-NaCl-haltigem Salzmedium (WT/4 und GK2/4) kultiviert. Für die Kultivierung der Zellen der Mutante GK2 wurden in einem Versuch Medien verwandt, die mit 1 mol/l Glucosylglycerol (GK2/0 + GG und GK2/4 + GG) versetzt waren (schraffierte Säulen).

Nach Identifizierung des Gens, das in *Synechocystis* für die Glucosylglycerol-Phosphat-Synthase (GGPS) kodiert, wurden Mutanten hergestellt, die ausschließlich in diesem Gen einen Defekt aufweisen. Das geschah auf gentechnischem Wege, indem in die kodierende Sequenz des *ggpS*-Gens eine Antibiotika-Resistenzgen-Kassette eingebaut wurde. Die entstandende Mutante wurde mit GK2 bezeichnet. Die Mutante GK2 unterschied sich hinsichtlich der Streßtoleranz klar von den Ausgangszellen des Wildtyps. Unter salzarmen oder Kontrollbedingungen wuchsen Zellen des WT (WT/0) und von GK2 (GK2/0) vergleichbar gut. Durch den Defekt im *ggpS*-Gen konnten salzbelastete Zellen der Mutante GK2 (GK2/4) jedoch kein GG akkumulieren (Abb. 2 A). Diese Unfähigkeit zur GG-Akkumulation führte dazu, daß die Photosynthese vollständig inhibiert wurde (Abb: 2 B), und die Zellen auf salzhaltigem Medien abstarben. Durch dieses Experiment konnte ein kausaler Zusammenhang zwischen einem intakten *ggpS*-Gen, wie in Zellen des WT vorliegend, und einer hohen Salztoleranz hergestellt werden.

In zusätzlichen Experimenten wurde gezeigt, daß nicht die Gensequenz direkt, sondern das durch die enzymatische Aktivität der GGPS synthetisierte GG für diese Schutzwirkung verantwortlich ist. Zellen von *Synechocystis* verfügen über ein Transportsytem, mit dem sie aus dem umgebenden Medium frei gelöstes GG in die Zelle transportieren können. Daher wurden in weiteren Experimenten Zellen der Mutante GK2 in Gegenwart von GG (Zusatz von 1 mM GG zum Medium) einem Salzschock ausgesetzt, die Ergebnisse sind mit gestrichelten Säulen dargestellt. Nunmehr sind die salzgeschockten Zellen der Mutante (GK2/4 + GG) in der Lage, GG durch Aufnahme aus dem Außenmedium im Inneren zu akkumulieren. Die auf diesem Wege erzielten internen Konzentrationen entsprechen denen, die Zellen des WT durch Synthese mit Hilfe der intakten GGPS erreichen (Fig. 2). Die hohe interne GG-Konzentration gestattet eine Anpassung des Photosyntheseapparates der Mutante GK2 (GK2/4 + GG) an die Salzbelastung (Abb. 2 B), so daß diese Mutantenzellen nunmehr auch unter diesen salzreichen Bedingungen Wachstum zeigen können. Durch den Zusatz von GG und dessen Aufnahme durch die Zellen konnte der Defekt des *ggpS*-Gens phänotypisch komplementiert werden.

## Patentansprüche

1. Verwendung eines Glucosylglycerol-phosphat-synthase-gens, welches für ein Protein mit einer Aminosäuresequenz codiert, die zu mehr als 60% identisch zu einer Aminosäuresequenz ist, die aus der in Tabelle 1 dargestellten DNA-Sequenz abgeleitet ist, zur Erzeugung von Glucosylglycerol in Kulturpflanzen.

2. Verwendung eines Proteins mit einer Aminosäuresequenz, die zu mehr als 60% identisch ist zu einer aus der in Tabelle 1 dargestellten DNA-Sequenz abgeleiteten Aminosäuresequenz, wobei das Protein Glucosylglycerol-phosphat-synthase-Aktivität besitzt, zur Erzeugung von Glucosylglycerol in Kulturpflanzen.

3. Verwendung eines Proteins gemäß Anspruch 2, wobei das Proteim zu mehr als 70% identisch ist zu einer aus der in der Tabelle 1 dargestellten DNA-Sequenz abgeleiteten Aminosäuresequenz.

4. Verwendung eines Proteins gemäß Anspruch 3, wobei das Proteim zu mehr als 80% identisch ist zu einer aus der in Tabelle 1 dargestellten DNA-Sequenz abgeleiteten Aminosäuresequenz.

5. Verwendung eines Proteins gemäß Anspruch 4, wobei das Proteim zu mehr als 90% identisch ist zu einer aus der in Tabelle 1 dargestellten DNA-Sequenz abgeleiteten Aminosäuresequenz.

6. Transgene Nutzpflanze, **dadurch gekennzeichnet, dass** sie ein übertragenes Glucosylglycerol-phosphat-synthase-gen trägt, welches für ein Protein mit einer Aminosäuresequenz codiert, die zu mehr als 60% identisch zu einer Aminosäuresequenz ist, die aus der in Tabelle 1 dargestellten DNA-Sequenz abgeleitet ist.

7. Transgene Nutzpflanze gemäß Anspruch 6, **dadurch gekennzeichnet, dass** sie spezifisch Glucosylglycerol anreichert, so dass sie eine Toleranz gegenüber Trockenheit, Kälte, hohen Bodensalzgehalt, oxidativen Stress oder Hitze aufweist.

8. Transgene Nutzpflanze nach einem der Ansprüche 6 oder 7, **dadurch gekennzeichnet, dass** sie zusätzlich ein Glucosylglycerolphosphat-phosphatase-gen trägt.

9. Transgene Nutzpflanze gemäß einem der Ansprüche 6 bis 8, **dadurch gekennzeichnet, dass** die Pflanze ausgewählt ist aus Nahrungs- und Futtermittelpflanzen und Pflanzen der nachwachsenden Rohstoffe.

10. Verwendung einer transgenen Nutzpflanze nach einem der Ansprüche 6 bis 9 zum Anbau unter Bedingungen eines Wassermangels, hohen Bodensalzgehaltes, oxidativen Stresses, Starklicht, Hitze oder Kälte.

## Claims

1. Use of a glucosylglycerol-phosphate synthase gene encoding a protein having an amino acid sequence that is more than 60 % identical to an amino acid sequence derived from the DNA sequence represented in Table 1 for producing glucosylglycerol in cultivated plants.

2. Use of a protein having an amino acid sequence that is more than 60 % identical to an amino acid sequence derived from the DNA sequence represented in Table 1, said protein displaying glucosylglycerol-phosphate synthase activity for producing glucosylglycerol in cultivated plants.

3. Use of a protein according to claim 2, wherein the protein is more than 70 % identical to an amino acid sequence derived from the DNA sequence represented in Table 1.

4. Use of a protein according to claim 3, wherein the protein is more than 80 % identical to an amino acid sequence derived from the DNA sequence represented in Table 1.

5. Use of a protein according to claim 4, wherein the protein is more than 90 % identical to an amino acid sequence derived from the DNA sequence represented in Table 1.

6. A transgenic plant, **characterized in that** it carries a transferred glucosylglycerol-phosphate synthase gene encoding a protein having an amino acid sequence that is more than 60 % identical to an amino acid sequence derived from the DNA sequence represented in Table 1.

7. The transgenic plant according to claim 6, **characterized in that** it specifically accumulates glucosylglycerol so that it is tolerant of drought, cold, high soil salt concentrations, oxidative stress or heat.

8. The transgenic plant according to any of the claims 6 or 7, **characterized in that** it additionally carries a glucosylglycerol-phosphate phosphatase gene.

9. The transgenic plant according to any of the claims 6 through 8, **characterized in that** the plant is selected from food and feed plants and from plants cultivated as renewable raw materials.

10. Use of a transgenic plant according to any of the claims 6 through 9 for cultivation under conditions including lack of water, high soil salt concentrations, oxidative stress, strong light, heat or cold.

## Revendications

1. Utilisation du gène de glucosylglycérolphosphate synthase codant pour une protéine composée d'une séquence d'acides aminés identique à plus de 60 % à une séquence d'acides aminés dérivée de la séquence d'ADN représentée au tableau 1 en vue de produire du glucosylglycérole dans des plantes cultivées.

2. Utilisation d'une protéine composée d'une séquence d'acides aminés identique à plus de 60 % à une séquence d'acides aminés dérivée de la séquence d'ADN représentée au tableau 1, la protéine possédant une activité de glucosylglycérole phosphate synthase, en vue de produire du glucosylglycérole dans des plantes cultivées.

3. Utilisation d'une protéine selon la revendication 2, la protéine étant identique à plus de 70 % à une séquence d'acides aminés dérivée de la séquence d'ADN représentée au tableau 1.

4. Utilisation d'une protéine selon la revendication 3, la protéine étant identique à plus de 80 % à une séquence d'acides aminés dérivée de la séquence d'ADN représentée au tableau 1.

5. Utilisation d'une protéine selon la revendication 4, la protéine étant identique à plus de 90 % à une séquence d'acides aminés dérivée de la séquence d'ADN représentée au tableau 1.

6. Plante utile transgénique, **caractérisée en ce qu'**elle porte un gène de la glucosylglycérole phosphate synthase codant pour une protéine composée d'une séquence d'acides aminés identique à plus de 60 % à une séquence d'acides aminés dérivée de la séquence d'ADN représentée au tableau 1.

7. Plante utile transgénique selon la revendication 6, **caractérisée en ce qu'**elle accumule spécifiquement du glucosylglycérole de sorte qu'elle est tolérante à la sécheresse, au froid, à la salinité élevée du sol, au stress oxydatif ou à la chaleur.

8. Plante utile transgénique selon l'une quelconque des revendications 6 ou 7, **caractérisée en ce qu'**elle porte en plus un gène de la glucosylglycérole phosphate phosphatase.

9. Plante utile transgénique selon l'une quelconque des revendications 6 à 8, **caractérisée en ce que** la plante est choisie parmi les plantes alimentaires et fourragères et parmi les plantes cultivées pour obtenir des matières premières renouvelables.

10. Utilisation d'une plante utile transgénique selon l'une quelconque des revendications 6 à 9, destinée à être cultivée dans des conditions de manque d'eau, de salinité élevée du sol, de stress oxydatif, de lumière intense, de chaleur ou de froid.


### SEQUENZ PROTOKOLL

<110> Norika-Nordring-Kartoffelzucht- und Vermehrungs Gm
<120> Glucosylglycerolphosphatsynthasegene zur Erzeugung der niedermolekularen Schutzsubstanz Glucosylglycerol und ihre Verwendung
<130> nok-1-98-pct
<140>
   <141>
<150> 198 32 334.4
   <151> 1999-07-17
<160> 1
<170> PatentIn Vor. 2.1
<210> 1
   <211> 1500
   <212> DNA
   <213> Synechocystis PCC6803
<400> 1
   atgaattcat cccttgtgat cctttaccac cgtgagccct acgacgaagt tagggaaaat 60
   ggcaaaacgg tgtatcgaga gaaaaagagt cccaacggga ttttgcccac cctcaaaagt 120
   ttttttgccg atgcggaaca gagcacctgg gtcgcatgga aacaggtttc gccgaagcaa 180
   aaggatgatt ttcaggcgga tatgtccatt gaaggccttg gcgatcgttg tacggtgcgc 240
   cgggtgcccc tgacggcgga gcaggtaaaa aacttctatc acatcacttc caaggaagcc 300
   ttttggccca ttctccactc tttcccctgg cagttcacct acgattcttc tgattgggat 360
   aattttcagc acattaaccg cttatttgcc gaggcggcct gtgccgatgc cgatgacaat 420
   gcattgtttt gggtccacga ctataacctc tggttagcgc ccctttacat tcgtcagctc 480
   aagcccaacg ccaagattgc ctttttccac cacaccccct tccccagcgt tgatattttc 540
   aatattttgc cctggcggga ggcgatcgta gaaagcttgc tggcctgtga tctctgtggt 600
   tttcatattc cccgctacgt agaaaatttt gtcgccgtgg cccgtagtct caagccggtg 660
   gaaatcacca gacgggttgt ggtagaccaa gcctttaccc cctacggtac ggccctggcg 720
   gaaccggaac tcaccaccca gttgcgttat ggcgatcgcc tcattaacct cgatgcgttt 780
   cccgtgggca ccaatccggc aaatatccgg gcgatcgtgg ccaaagaaag tgtgcaacaa 840
   aaagttgctg aaattaaaca agatttaggc ggtaagaggc taattgtttc cgctgggcgg 900
   gtggattacg tgaagggcac caaggaaatg ttgatgtgct atgaacgtct actggagcgt 960
   cgccccgaat tgcaggggga aattagcctg gtagtccccg tagccaaggc cgctgaggga 1020
   atgcgtattt atcgcaacgc ccaaaacgaa attgaacgac tggcagggaa aattaacggt 1080
   cgctttgcca aactgtcctg gacaccagtg atgctgttca cctctccttt agcctatgag 1140
   gagctcattg ccctgttctg tgccgccgac attgcctgga tcactcccct gcgggatggg 1200
   ctaaacctgg tggctaagga gtatgtggtg gctaaaaatg gcgaagaagg agttctgatc 1260
   ctctcggaat ttgccggttg tgcggtggaa ctacccgatg cggtgttgac taacccctac 1320
   gcttccagcc gtatggacga atccattgac caggccctgg ccatggacaa agacgaacag 1380
   aaaaaacgca tggggagaat gtacgccgcc attaagcgtt acgacgttca acaatgggcc 1440
   aatcacctac tgcgggaagc ctacgccgat gtggtactgg gagagccccc ccaaatgtag 1500